# EUROPEAN PATENT SPECIFICATION

(11) **EP 2 564 840 B1**
(45) Date of publication and mention of the grant of the patent: **26.11.2014**
(21) Application number: 10724102.8
(22) Date of filing: 26.04.2010
(51) Int. Cl.: A61K 31/19, A61K 31/20, A61K 31/23, A61K 36/8962, A61P 1/04, A61K 36/9066, A61K 9/28

(54) **COMPOSITIONS FOR THE SYMPTOMATIC RELIEF OF STOMACH PAIN OR GASTRO-OESOPHAGEAL REFLUX**
ZUSAMMENSETZUNGEN FÜR DIE SYMPTOMATISCHE LINDERUNG VON MAGENSCHMERZEN ODER GASTROÖSOPHAGEALEM REFLUX
COMPOSITIONS POUR L'ATTÉNUATION SYMPTOMATIQUE DES DOULEURS D'ESTOMAC OU DU REFLUX GASTRO-OESOPHAGIEN

(43) Date of publication of application: 06.03.2013
(73) Proprietor: Gago De Santos, Angel Manuel, 28035 Madrid (ES)
(72) Inventor: Gago De Santos, Angel Manuel, 28035 Madrid (ES)
(74) Representative: Elzaburu Marquez, Alberto
(86) International application number: PCT/ES2010/070261
(87) International publication number: WO 2011/135107

(56) References cited:
- EP-A1- 0 702 002
- WO-A1-03/074042
- US-A- 5 989 560

## Description

### Field of the Invention

The invention refers to new useful compositions to alleviate symptoms of what is commonly known as stomach acidity, heartburn or, more technically, esophageal reflux (a very generic term that encompasses a large number of symptoms).

### Background of the Invention

The term commonly known as chronic heartburn or stomach acidity is often the most common manifestation of a clinical condition referred to as esophageal reflux, also known as GERD or GORD (Gastro-Esophageal Reflux Disease or Gastro-Oesophageal Reflux Disease). Among other things, GERD is characterized by a series of symptoms such as heartburn, chronic indigestion (dyspepsia), pyrosis, abdominal pain and swelling, epigastric pain, nausea, regurgitation, premature feeling of satiety, acid reflux coughing, vomiting and gas indigestion.

GERD is a disease that mainly consists of abnormal refluxing of gastric contents into the esophagus through the lower esophageal sphincter (LES). The LES is the lowest portion of the esophagus where it joins the stomach. Functionally, it maintains the esophageal pressure at a level greater than the intragastric pressure, thus preventing the reverse passage of gastric content. LES is prone to intermittent relaxation. When this occurs, the physical barrier separating both organs is lost, and the stomach fluid can return to the esophagus. The belief is that the reflux is due to the larger number of LES relaxations, which allows the gastric contents to back up into the esophagus repeatedly. The pH of the gastric content is acid, which is why the feeling of *heartburn* is produced (in the pit of the stomach) and, when it reaches the throat or the mouth, the taste is acidic and bitter, a symptom known as pyrosis. Burning and pyrosis are common symptoms, but they do not necessarily mean there is GERD. The symptoms may only be considered GERD if they recur. Anyone can suffer from the disease.

This document refers to "gastroesophageal disease" or "GERD" as the manifestation of any of the symptoms mentioned above, as defined by Van Heerwarden, M. A., Smout A. J. P. M., 2000; Diagnosis of reflux disease. Baillière's Clin. Gastroenterol. 14, pp. 759-774. This covers all forms and manifestations of GERD, including erosive and non-erosive GERD, heartburn and all other symptoms associated with the general term, GERD. In all its manifestations and derivations, GERD is presently the most prevalent disease in the upper digestive system, and it is very prevalent in the Western world. It is estimated that at least 30 million patients suffer one of the aforementioned forms of GERD in the US each year, with the number rising significantly every year. Around 7% of Americans suffer heartburn every day, and 14% at least once every week. Other estimates suggest that around 20% of the US population suffer these symptoms. The greatest incidence occurs in adults over forty.¹

There is currently literature on some compositions for the treatment of GERD, such as the ones described in the following documents:

Document US2009208588 (A1), which describes a dietary supplement composed of essential sugars and honey as natural alternatives to medication, specifically to proton pump inhibitors, used for treating GERD.

International publication WO2009113594 (A1) describes the use of mixtures of glutamic acid and arginine, or derivatives thereof, for gastrointestinal disorder prophylaxis and relief. These mixtures seem to be especially effective against upper digestive tract disorders, and particularly dyspepsia, bloating, heartburn and gastroesophageal reflux or GERD.

Document KR20090048393 (A) describes the use of a specific group of polysaccharides to reduce the symptoms of heartburn and gastroesophageal reflux. Among these polysaccharides, galactomannans and glucomannans are mentioned among others as having high potential for hydration, high viscosity, and high stability at low pH.

International publication WO2008091170 (A1) describes a matrix that contains an oral sodium butyrate preparation protected in a matrix of triglycerides and Yucca Schidigeri extract, for the treatment and prophylaxis of inflammatory diseases of the large intestine and colorectal cancer.

However, none of these compositions has proven the effectiveness in the relief of GERD symptoms, with absence of secondary effects, demonstrated by the compositions of the invention.

Consequently, there is still the need for alternative compositions that relieve GERD symptoms while at the same time reducing or minimizing their side effects.

The solution to this problem is based on the fact that the inventors of the present invention have discovered that it is possible to mitigate GERD symptoms by combining agents with anti-inflammatory, trophic, antimicrobial, humoral and genetic-expression-regulating properties that do the following:
a) allow the reduction of the inflammation due to chemo-enzymatic damage, thus reducing pain and irritation;
b) act as trophic agents promoting better and greater epithelial regeneration of the damaged tissue, thus avoiding the chemical damage indicated in point a);
c) are regulators of genetic expression, thus reducing the probabilities of abnormal replications that result in anaplastic tissue formation;
d) improve digestion in general, especially better enzymatic digestion of food, by inducing an increased production of digestive enzymes, both at pancreatic as well as at small intestinal epithelial levels, thus avoiding the possible problems caused by inefficient digestion of food, or its poor absorption, and
e) promote beneficial endogenous flora, whether directly, by serving as trophic factors for the development of strains of bacteria that the scientific community may consider as beneficial for the host, or indirectly, through its well-known antimicrobial, anti-yeast and antifungal activities.

Generally, the combinations herein disclosed comprise agents from the following two groups:
a) short- and medium-chain fatty acids (SCFA and MCFA), especially butyric, iso-butyric, caproic, caprylic and capric acids, in any combination, as well as their derivatives, particularly their esters. One of the specifically preferred SCFA is sodium butyrate (CH₃-CH₂-CH₂-COO⁻Na⁺), and especially buffered sodium butyrate. In the present document, "short chain fatty acid" shall be understood as a fatty acid with 1 to 4 carbon atoms, that is a C1-C4 fatty acid. Also, "medium-chain fatty acid" shall be understood as a fatty acid with 5 to 10 carbon atoms, that is, a C5-C10 fatty acid.
b) Alliaceae extracts, and especially garlic, which are all rich in organosulfur compounds (OSC), or curcuma root extracts rich in curcumin, given that their anti-inflammatory, antibacterial and antitumor properties have already been widely described. Special attention is paid to garlic powder, which is preferably not deodorized so that its organosulfur compound (OSC) content is as high as possible.

Both groups of compounds are known separately, and are authorized for human and animal consumption. According to the American FDA, specifically, butyric acid, its sodium salt, sodium butyrate, and its glycerol triester are GRAS (Generally Regarded As Safe) compounds. Obviously, garlic, curcuma and, in general, alliaceae are also included. However, to the knowledge of the inventor, no description has been made of combinations of both groups of compounds until now, much less combinations that render the beneficial effects produced by the compositions of the invention.
Consequently, a first feature of the invention is directed to compositions that comprise:
- at least one C1-C10 fatty acid or any derivative thereof, and
- a plant extract from the alliaceae family,
in line with the scope of attached claim 1.

All the compositions are indicated as dry matter, except if otherwise stated.

In the compositions of the invention, the C1-C10 fatty acid is preferably at a concentration of 25 to 95%, more preferably 35 to 80%, and most preferably 50 to 60%, of the weight of the initial composition. This fatty acid may be in the form of the acid or any derivative thereof, particularly salts or esters thereof.

Also in the compositions of the invention, the plant extract from the alliaceae family is preferably at a concentration of 5 to 30%, more preferably 5 to 25%, and most preferably 5 to 15%

The compositions of the invention are generally produced in the form of capsules, tablets, sachets, pills or coated pills. The product of the invention is preferably administered orally.

A second feature of the invention is directed to the use of these compositions as a dietary supplement.

A third feature of the invention is directed to using these compositions as medication, and specifically as medication for re-establishing digestive homeostasis affected by peptic acid disorders and/or conditions caused by damage to the gastric mucosa or the digestive epithelium, and, more particularly, for relieving GERD symptoms.

The inventor has found, surprisingly, that the compositions according to the invention described herein are particularly effective in mitigating heartburn as the most common symptom of GERD. The surprise was even greater, since countless clinical manuals and publications advise against the consumption of alliaceous plants because this can help increase LES relaxation. On the other hand, no scientific publication makes the connection between short and medium chain fatty acids and GERD, although a great deal of research work exists on their positive effects in the lower digestive tract of animals, including human beings.

Until now, scientific publications have failed to describe the effect these compounds bear on the symptoms listed in this document. It should be mentioned that many consider garlic as a compound that aggravates dyspepsia symptoms², stomach and intestinal discomforts, and that chronic patients suffering from GERD or its variants are often advised to remove it from their diets. With no intention of being bound by the theory, the belief is that the mixture of both products, swallowed in the form of capsules, is released in the stomach. Both the organosulfur compounds (OSC) of the alliaceae as well as the butyrate itself (and the SCFA) are volatile compounds. During LES relaxation, volatile compounds are able to reach the distal area of the esophagus, long before the acid reflux does. It is in this area of the esophagus that the gastric juice causes more damage to the epithelium. Unlike that of the stomach, the epithelium of the esophagus is not protected by a layer of protective mucus, and it is therefore subject to a greater chemical and enzymatic aggression from reflux gastric juices. Upon reaching the epithelium, the active compounds of the invention play a key metabolic role in dynamically keeping the cell wall of the digestive tract intact, thus helping to restore the repair mechanisms that seem to be essential in preventing and resolving inflammatory processes. By encouraging faster epithelial regeneration, the integrity of the epithelial barrier becomes more effective, reducing the effect of the gastric juices on the inner layers of the esophagus epithelium or submucosa. Thus the continuous erosion of the esophagus is mitigated and reduced, and the chemical aggression contained. Though preventing relaxations and reflux is still impossible, a new dynamic of epithelial regeneration has been established to face chemical damage.

On the other hand, rapid epithelial regeneration leads to rapid and constant cellular mitosis and, therefore, cellular DNA replication. Thanks to the modulating properties of genetic expression (HDI's), butyrate and organosulfurs of the alliaceae prevent abnormal and aberrant replications, thus maintaining a normal and healthy epithelium.

It is known that garlic organosulfur compounds inhibit the growth and replication of *Helicobacter Pylori.* Although not all GERD or peptic ulcer patients suffer infections caused by this bacteria, its presence aggravates the histopathological damage produced by acid reflux. In cases in which a patient is infected by the bacteria, administering the garlic from the product will mitigate the effects³.

### Detailed Description of the Invention

The current state of the art does not seem to provide satisfactory solutions for the disease, since it is not known how to effectively prevent the inappropriate relaxations of LES, thus rendering it a chronic condition, not exempt from important complications. Stomach acid production is a natural process that is necessary in the normal digestion of food. Not only does it permit the creation of an environment conducive to maximum activity of digestive enzymes, but it also plays an important role as prophylactic barrier, preventing the entry and multiplication of pathogens ingested by mouth (bacteria, fungi, yeast and viruses). If, as the latest studies suggest, GERD patients do not actually suffer from stomach hyperacidity (Holloway & Dent, 1990, Gastroenterol. Clin. N. Amer. 19, pp. 517-535), rather their production levels are normal, it is reasonable to propose a new maintenance therapy that involves reducing, repairing and preventing, at the cellular level, damage caused by reflux due to transient relaxations of the LES.

LES remains generally dosed after a meal. During relaxation it permits the reflux of acid and/or food particles towards the esophagus. Gastric distension induced in a significant number of GERD patients for experimental purposes increased the number of transient LES relaxations, the main cause of reflux episodes. The higher the number of transient LES relaxations, the higher the frequency of the episodes, such that the cumulative time the acid material is in contact with the esophageal mucosa increases. Pathological reflux patients often suffer from many short-lived and/or several prolonged reflux episodes during which the acid can remain in the esophagus for several hours.

Ineffective esophageal peristalsis is another factor that increases the time the esophagus of GERD patients is exposed to acid. Even though peristalsis occurs, the wave generated is ineffective because of the diminished amplitude of secondary peristaltic waves. Although the length of time the esophagus is exposed to the acid correlates with the frequency of symptoms and with the degree and severity of the esophageal mucosa damage, the degree of esophageal mucosa damage can markedly increase if the luminal pH is less than 2, or if pepsin or conjugated bile salts are present in the reflux. The stomach uses various mechanisms to protect itself from excess acid and proteolytic enzymes. Constant dynamism exists between the pepsin-acid chemical aggression and the self-defense of the mucosa. Normally, the mucosa withstands the chemical and enzymatic attack while maintaining its integrity and health. Parietal cell walls and the main cell walls of the gastric mucosa are highly impermeable to acid. Other protection mechanisms are as follows:
a) a layer of alkaline mucus containing bicarbonate, which covers the gastric mucosa;
b) the presence of strong and intimate bonds between adjacent epithelial cells prevents filtration of acid down to the submucosa;
c) constant cell replication that permits the effective, uninterrupted replacement of damaged cells, and
d) the production of prostaglandins, which inhibit and regulate gastric secretion. Prostaglandins increase blood flow in the mucosa, stimulating bicarbonate and mucus secretion. Notwithstanding, excessive acid production or any other failure in the mucosa protection system can destroy this delicate defense balance. Chemical damage forms ulcers, like holes in the mucosa, thus letting the gastric juice filter down to the submucosa. The subsequent cell damage triggers the inflammatory mechanisms of cells (gastritis).

The histopathological damage associated with GERD includes distal esophagus inflammation (esophagitis), erythema, isolated erosions, confluent erosions, circumferential erosions, deep ulcers, replacement of normal esophageal epithelium with intestinal epithelium (Barrett's esophagus), in which it is very common that the new epithelium degenerates into neoplasia and anaplasia (cancer). Other indirectly linked damage is: pulmonary aspiration, chronic coughing, and laryngitis. Reflux is often asymptomatic in the absence of esophagitis. Pyrosis is the most frequent symptom, but acid regurgitation, dyspepsia, thoracic pain and dysphagia can also appear. Hemorrhages, pharyngitis, laryngitis and, due to the aspiration of gastric contents, bronchospasms like asthma attacks, aspiration pneumonias or even pulmonary fibrosis can also appear. All these altered states of the digestive system are characterized by alterations in gastric motility, sensitivity and secretion, as well as by a greater incidence of *Helicobacter pylori* infections.

Hiatus hernia is a condition that predisposes to reflux. This happens when part of the stomach slides through the esophagus hiatus (a hole in the diaphragm through which the esophagus slides to the abdomen) from the abdominal cavity to the thorax. The abnormal localization of the stomach puts external pressure on the gastric content, which promotes reflux. In the absence of hernia, the LES and esophageal hiatus are aligned and somehow the latter reinforces LES; this relationship is lost in the hiatus hernia.

It is accepted that the most important cause of reflux depends on a hypotonic LES, although more detailed studies (Holloway & Dent [1990] Gastroenterol. Clin. N. Amer. 19, pp. 517-535) have shown that most acid reflux episodes occur during transient relaxations of LES (TRLES) or "lower esophageal sphincter relaxations" (TLESR), that is, relaxations not triggered by deglutition. Clinically, the uncontrolled release or continuous hypersecretion of acid can provoke changes in the gastric and intestinal epithelium, often causing severe esophageal erosions of the metaplasic-kind, with high risk of cancer and death. The most recent studies indicate that long recurrent periods of gastric hypersecretion can degenerate into cancer formations. However, contrary to what is commonly accepted, certain studies point out that the level of acid secretion is normal in patients diagnosed with GERD. This seems to indicate that if hypersecretion is not the main cause in the histological changes observed, it could be the repeated and continuous aggression of the gastric content that refluxes as a result of the loss of motility in the LES.

Current pharmacotherapy often employs three strategies, listed from the newest to the oldest:
1- Reducing acid secretion in the stomach using Histamine-2 antagonists or proton pump inhibitors;
2- Preventing the stomach contents from rising up to the esophagus by creating a foam layer in the reflux interphase. These products are known as foaming agents;
3.- Increasing gastric motility and facilitating pressure reduction on the LES, thus reducing reflux (metoclopramide, cisapride, bethanechol, domperidone). Cisapride was withdrawn from the American market for safety reasons.
4- Or, if not available, its neutralization, either in the stomach or in the esophagus using basic substances known as antacids.

From the three strategies outlined above, the first, which consists in reducing acid secretion, is the newest. This can be controlled or reduced in three ways:
a) Histamine receptor inhibition in the basolateral membrane of parietal cells. Among others cimetidine, famotidine, nizatidine and ranitidine are found.
b) Stomach proton pump inhibition. These medications are known as proton pump inhibitors or PPIs. Among others are omeprazole, lansoprazole, pantoprazole, rabeprazole and esomeprazole. Proton pump inhibitors irreversibly block the ATPase (H⁺/K⁺ ATPase) enzyme in the membrane, which interchanges hydrogen for potassium on both sides of the lipid bilayer of the cell, also referred to as proton pumps. This enzyme participates in the terminal stage of proton secretion in the stomach; it is directly responsible for the secretion of H⁺ ions to the stomach lumen.

Histamine 2 (H2) receptor inhibitors are not as effective in the treatment of this pathology as PPIs. The use of PPIs in the final stage of acid release as target, as well as the irreversible nature of the inhibition, renders these kinds of medications more effective than H2 antagonists. Acid secretion is reduced by between 90-99% for 24 hours. The standard therapy often consists of the administration of PPIs for a short period of time (2-6 weeks) to reduce gastric secretion and to permit regeneration of the damaged epithelium, as well as scar tissue formation at the possible erosions or ulcers.

Both therapies have clearly improved the quality of life of patients who suffer from this disease; nonetheless, a great number of patients have suffered recurrent relapses after treatment. Relapses have also been found to occur during treatment. In spite of its high efficiency rate and its generalized worldwide usage, it has been shown that PPIs are not absolutely effective in many cases. It is estimated that about 30% of GERD patients maintain the symptoms with the standard dosage of PPI. The plasma half-life of PPI is short, leading many patients undergoing treatment to suffer acid episodes at night. The effective therapeutic levels for PPIs are gradually reached after doses continue for 4-5 days. This may be explained by the fact that only PPIs are capable of exclusively blocking pumps that are active when the medication is present. After they are administered, the stomach gradually returns to its normal acid secretion, mainly through *de novo* synthesis of H⁺/K⁺ ATPase enzyme.

It has been reported that the symptoms of erosive esophagitis studied in certain advanced clinical studies on gastro-esophageal reflux tend to recur in more than 80% of the patients within 6 months after the administration of proton pump inhibitors is discontinued. It is therefore common practice in the medical community to administer long-term maintenance therapy with full or half dosage, to inhibit recurrence or other complications of the disease. This new strategy of generalized use of PPI's is not devoid of complications in terms of effectiveness, tolerance, convenience and safety.

We should mention the precancerous condition known as Barret's Esophagus (BE) as an example of the importance of the chronic nature of this disorder. BE often develops from long and prolonged GERD because the continuous chemical and enzymatic attacks on the epithelium of the esophagus culminate into abnormal cell degeneration. BE is one of the most important precursor states in esophageal adenocarcinoma (EAC). The risk that patients diagnosed with BE develop EAC is 30 to 60% more than in the general population. Over the last two decades, EAC incidence has drastically increased in most Western countries and it is currently the cause of at least 60% of esophageal cancer cases. In addition to said EAC risk, BE has also been associated with a greater risk of colorectal cancer (CRC). In 1985, about 5.5% more cases of malignant colorectal neoplasias were reported in BE patients, significantly above what could be expected within a normal population. However, several studies have since published contradictory results, with some confirming the data while others failed to do so. These discrepancies may be due to the low number of BE patients in said studies, the lack of histological confirmation of endoscopic diagnosis of BE, bias in the diagnosis, use of an inappropriate control group, or even the absence of a control group. Although a more precise clarification of this link may be necessary to confirm the development of BE and the appearance of CRC, there are indications that GERD may be the initial condition that increases the risk of developing these types of severe complications: EAC and CRC.

In specific embodiments of the invention, the short- or medium-chain fatty acid of the compositions of the invention are butyric acid or isobutyric acid, alone or in any combination thereof, as well as salts and esters thereof, and more specifically glycerol esters thereof, in any combination. In cases when salts are used, in preferred embodiments of the invention these salts are alkali or alkaline-earth metal salts, especially sodium (Na), calcium (Ca) or potassium (K), and they are preferably buffered prior to adding the plant extract. The buffer is preferably selected from phosphoric (sodium-potassium phosphates) and carbonic (sodium-potassium carbonate-bicarbonate) salt mixtures. Examples of phosphate salts are trisodium phosphate (Na₃PO₄), disodium phosphate (Na₂HPO₄) and monosodium phosphate (NaH₂PO₄). Examples of carbonate salts are sodium carbonate (Na₂CO₃) and sodium hydrogen carbonate (NaHCO₃). The proportions of buffer salts may vary since they are the result of the acid-base reaction between a liquid (butyric acid) and a solid mixture of, for example, anhydrous trisodium phosphate, disodium phosphate dodecahydrate, sodium carbonate.

The plant extract from the alliaceae family in the compositions of the invention is preferably vegetable garlic extract and, more preferably, powdered, dry garlic that is not deodorized. However, it is also possible to use other forms of garlic, such as oil, aged garlic, etc., in the compositions of the invention.

Optionally, the compositions of the invention also comprise curcuma plant extracts, preferably powdered curcuma with high curcumin content, preferably with a concentration between 5 and 25%, more preferably between 5 and 15%, and most preferably between 5 and 10% of the initial composition. Nevertheless, in the same manner as in the case of garlic, it is also possible to use other forms, such as oil, etc., of curcuma plant extracts in the compositions of the invention.

Also optionally, the compositions of the invention may include an excipient acceptable for nutritional purposes. Examples of such excipients are thinners, disintegrants, lubricants, binders, gellants, flavors, sweeteners, colorants, preservatives, fillers, antioxidants, encapsulating coadjuvants and coating agents.
The compositions of the invention may be prepared using standard mixing techniques, just like conventional pharmaceutical excipient compounds. Examples of vehicles and formulations are capsules, pills, coated pills, sachets, granules and, in general, any method possible for oral administration. The material must be protected from humidity, which is why it is preferable to use in capsules, pills or coated pills that permit easy swallowing, and that easily dissolve in the stomach. Coadjuvants authorized by the pharmacopeia like stearate, cellulose, lactose and silica may be added for their encapsulation. Stearate is specifically used because it has been proven to be the most suitable coadjuvant for encapsulating the product of the invention.

Examples of solid vehicles that may be used in the product of the invention are all those that are often used in the manufacture of pills, tablets and capsules, like lactose, starch, glucose, methylcellulose, magnesium stearate, dicalcium phosphates, mannitol and silica, among others. The compositions of the invention may be coated with one or more layers to reduce or prevent their characteristic odor. This coating may be made up of any product commonly used in the medication and supplement industries, like sugar, fats, biodegradable polymers, etc.

The compositions of the invention may be presented in any medium that facilitates their oral administration. The preferable form of presentation of the compositions of the invention is capsule, pill, sachet, tablet or coated tablet.

A preferred composition of the invention based on the general formula above could be the following:
- Butyric acid buffered with phosphate and/or carbonate salts: from 50% to 90% by weight of the total weight of the initial composition. Examples of phosphate salts are trisodium phosphate (Na₃PO₄), disodium phosphate (Na₂HPO₄) and monosodium phosphate (NaH₂PO₄). Examples of carbonate salts are sodium carbonate (Na₂CO₃) and sodium hydrogen carbonate (NaHCO₃). The proportions of buffer salts may vary as a result of the acid-base reaction between a liquid (butyric acid) and a solid mixture of anhydrous trisodium phosphate, disodium phosphate dodecahydrate and, optionally, sodium carbonate.
- Medium-chain fatty acids: 5 to 10% by weight of the total weight of the initial composition, especially like mono-, diglyceride and triglyceride esters;
- Garlic or its extracts: 5 to 20% by weight of the total weight of the initial composition, especially dehydrated and non-deodorized garlic powder; and optionally
- Curcuma: 5 to 20% by weight over the total weight of the initial composition in curcuma extract form, preferably with high curcumin content.

The compositions of the invention are useful to relieve the symptoms of heartburn and/or GERD, and specifically for manufacturing food supplements that may help to re-establish the digestive homeostasis affected by peptic-acid disorders and/or repair states due to damage done to the gastric mucosa or to the digestive epithelium.

### Butyric acid

Butyric acid is one of the Short-Chain Fatty acids (SCFA). Butyric acid, or its anion known as butyrate, can be represented, like any molecular entity with the formula CH₃-CH₂-CH₂-COO⁻ X⁺ where X⁺ may be any positively charged molecule or cation. In chemical terms, butyric acid would be defined as the unsaturated alkyl monocarboxylic acid with a straight four carbon chain. Butyrate is the negatively charged anion of said acid. The counter-anion or cation can be any positively charged chemical entity; specifically in the present invention, the most preferred counter-anion is the sodium salt, although, in principle, any other cation could be used for the purpose of the invention. In the present document, the terms butyrate and butyric acid are completely interchangeable.

Butyrate has been widely studied for over 60 years, although by 1878 scientists had already discovered that it existed in human feces. It is one of the most important fermentation acids in ruminants. Butyrate contains very interesting properties and its activity in mammalian biochemistry is important and varied. Butyrate is naturally produced in the body through catabolic oxidation of acetyl-CoA dependent on saturated long-chain fatty acids (Lehninger et al., 1993; Widmer et al., 1996). Generally, and from the biochemical and biological points of view, butyric acid is one of the most important metabolites in the "anaerobic fermentation" of carbohydrates in the digestive tract (mainly cecum and colon) in higher vertebrate living beings and, in particular, monogastric mammals, including man. Average concentrations of 20 mMol have been measured in human colon lumen.

Butyrate and other short-chain fatty acids are generated in the intestine by the carbohydrate bacterial metabolisms that resist digestion in the small intestine. These can be starch or simple carbohydrates that are not digested, pectins, hemicellulose or any polysaccharide, or protein that may be enzymatically degraded by the endogenous flora in the distal intestinal tract (Cummings, 1981; Bugaut and Bentejac, 1993; McIntyre et al., 1993; McIntosh et al., 1996; Whiteley et al., 1996). This partial degradation of the organic material that reaches the colon is, known as "fermentation", although the term is not used in the strict sense.

It is commonly accepted that the SCFA are the main source of energy for the colon and other related tissues. Out of the whole SCFA, acetic, propionic and butyric acids make up between 90-95% of all the SCFA in the colon⁴. These fermentation products in the colon of humans are the same as the cecum in herbivores and the rumen in ruminants⁵. SCFA deficiencies have been associated with colonic mucosa inflammations⁶, and with ulcerative colitis and other disorders⁷. Some studies have shown that increases in levels of SCFA leads to significant therapeutic improvements in areas such as the prevention of colorectal cancer⁸ and in the treatment of colitis⁹, and diverticulitis (Soergel, op. cit.). SCFAs are involved in the immune system and its response¹⁰, apoptosis in hepatic tumors¹¹; and in inhibiting the loss of fluids in mammals infected with cholera¹². In most of the studies cited, SCFAs were administered in the form of soluble salts: alkali metal acetates, propionates and butyrates. The administration of SCFAs, and mainly butyrate, either orally or through an enema or suppository, has shown to be effective in relieving the disorders mentioned above.

Many aspects of intestinal function and health depend on the appropriate levels of SCFA in the lumen. Although not all the beneficial effects of SCFA have been completely elucidated, the number of publications on the properties of butyrate in monogastric mammals and, specifically, in humans, may be classified into 6 groups:
**1.- Prebiotic**: Studies carried out in the 90s on pigs¹³ established the bases for elucidating the mechanism of action of butyrate on intestinal flora. A subsequent *in vivo* study on weaned piglets, Galfi & Neogrady (1991 and 1996), verified that increasing butyrate concentration in feed increased the lactobacilli count and reduced the *E*. *Coli* count, in all the intestinal tract sections studied. The authors therefore concluded that butyrate exercised a selective antimicrobial effect. The microbes were classified according to their sensitivity to butyrate: (1), Microbes sensitive to butyrate: *Clostridium acetobutylicum, E. coli, Streptococcus cremoris, Lactococcus lactis, L*. *cremoris,* and *Salmonella spp.;* (2) Microbes less sensitive to butyrate and pH independent: *Lactobacillus spp.* and *Streptococcus bovis.* Of all the SCFAs, butyric acid is the acid with the highest inhibition rate as the pH in the medium reduces.
   Therefore, SCFAs, and butyrate in particular, act by modulating the intestinal flora, since:
   a) On the one hand, they inhibit the growth of certain microorganisms like *E. Coli* and *clostridium* that are often damaging to the hosts.
   b) On the other, they favor the growth and replication of microorganisms that are beneficial for the intestinal chemism, such as lactobacillus. These effectively replace the void created by the reduction of the microorganisms sensitive to butyrate.
   c) The proliferation of lactobacilli strains helps reduce the pH of the medium, since they often produce lactic acid as catabolite in their metabolism. The inhibition effect of SCFAs and, in particular, butyrate, increases as the pH reduces. The butyric acid concentration necessary to reduce the *in vitro* count of cultures of microorganisms sensitive to butyrate reduces exponentially as the pH in the medium reduces. Galfi & Neogrady (1991 and 1996).
**2.- Energetic and Trophic:** Luminal butyrate is connected to the regeneration and differentiation of colonocytes, since they preferably use SCFAs and, butyrate in particular, as energy substrates. Colonocytes use butyric acid as their main source of energy, since it has been verified that, in its absence, they suffer apoptosis. Butyrate metabolization in colonocytes involves direct trophic action, as it is the engine that permits rapid cell replication. This rapid cell replication translates into significant changes at the histological level: intestinal villi grow, with much larger villi and much deeper crypts. (J Anim Sci. 2007 May; 85(5): 1184-91. Epub 2007 Feb 12). It has also been verified that butyrate also has a trophic action on the wall of the digestive tract.¹⁴
   In addition to colon cancer, butyrates have been studied for the treatment of intestinal inflammatory diseases, such as colitis and Crohn's disease. Butyrates increase the synthesis of colon mucin, a glycoprotein present in the colon mucus. The mucus sticks to the colon epithelium, thus preventing bacteria from invading the colon and protecting it against the target effect of toxins and bacterial enzymes. Butyrate enemas are used in the treatment of diverticulitis and ulcerative colitis¹⁵.
**4.-_Enzymatic:** The intravenous administration of SCFAs and, specifically, butyrate and isobutyrate, increases exocrine pancreatic secretions; in particular, amylase secretions. This cause-effect relationship depends on the concentration of the SCFAs administered¹⁶. The results of similar work done on new-born ruminants, which behave as monogastric animals, suggests that SCFAs may alter the flow of pancreatic juices through pH dependent and pH independent mechanisms localized in the proximal small intestine mucosa.
   Pancreatic juice enzymes are responsible for the digestion of complex carbohydrates, the hydrolysis of dietary proteins, helping to achieve better digestion and absorption of nutrients. This enzymatic effect, in synergy with the trophic effect of SCFAs, permits the food that has been digested better to be rapidly absorbed by the intestinal villi in the small intestine. Better absorption of food implies less partially undigested food in the large intestine (cecum and colon) thus preventing abnormal fermentations in the colon, which could cause an abnormal growth of the endogenous flora, as well as the production of biogenic amines (putrescine, cadaverine, scatol, histamines, among others); products with a marked inflammatory and teratogenic effect.
**5.- Regulation of genetic expression:** Central protein histone acetylation and deacetylation of nucleosomes in chromatin play a fundamental role in the regulation of genetic expression. (Gray and Ekstrom, 2001; Khochbin et al., 2001; Urnov, 2003). The state of histone acetylation is mainly controlled by two types of enzymes: histone acetyltransferases (HATs) and histone-deacetylases (HDAC). A series of HDAC inhibitors, known as HDIs, that reduce growth, differentiation and apoptosis in cancer cells, have been identified (Gore and Carducci, 2000; Marks et al., 2000; Weidle and Grossmann, 2000). HDIs are composed of a series of compounds of heterogeneous origin among which SCFAs, hydroxyamic acids, cyclic tetrapeptides and benzamides are included. Sodium butyrate is classified as an HDI. The treatment of cells with butyrate establishes their histones in acetylated form.

Sodium butyrate acts as an inhibitor against the growth of cancerous cells, having been attributed the capacity to induce the interruption of the cell cycle, differentiation and apoptosis (Medina et al., 1997; Richon et al., 1998; Wang et al., 1999; Butler et al., 2000). Several published studies have indicated that butyrate can modulate NF-κB transcription factor activity in various types of cells, including cancer lines of colon cells, isolated cells of the lamina propria, and macrophagi. The ability of butyrate to modulate NF-κB activity can be owed to its capacity to inhibit HDACs. Said capacity to modulate the NF-κB (nuclear factor kappa-light-chain-enhancer of activated B cells) activity makes this molecule an anti-cancer and anti-inflammation agent, since the NF-κB regulates the genes involved in the control of cell proliferation, cell death, immune response and inflammatory response. NF-κB is regulated through binding to inhibiting molecules generally known as IκBs (κB. inhibitors). There is a series of IκBs, including IκB-α, IκB-β, IκB-ε, p105, and p100. NF-κB activity often requires phosphorus-dependent ubiquitination and subsequent proteasomal degradation of one or several IκB molecules. There is evidence showing that butyrate prevents the entry of NF-κB initiated by the TNF-a (Tumor Necrosis Factor-alpha) into the cell nucleus as a result of its suppressor action on the proteasome activity. Butyrate influences the cell in a manner similar to a new class of anti-inflammatory and anticancer agents known as proteasome inhibitors.

The epithelial cells of the digestive tract form a translucent layer between the reducing medium of the lumen (translucent space through which food passes) and the oxidizing medium of the *lamina propria.* Cell metabolisms involved in the barrier functions of epithelial cells include substrate degradation, the capacity of forming new cell membranes, the detoxification of toxic agents present in the lumen, as well as the mechanisms that permit redox (reduction-oxidation) balance to be maintained between the two opposite media. Epithelial damage due to constant physiological and pathological offences may result in excessive exposure of the immune system of the host to antigenic, toxic and immunomodulating agents.

SCFAs play an important role in the reduction of intestinal inflammation: butyrate is effective in the treatment of certain inflammatory conditions affecting the distal digestive tract. Scientific studies propose butyrate as an anti-inflammatory agent in some inflammatory states of the digestive mucosa. The anti-inflammatory mechanism of this SCFA is a result of the modulation of certain proinflammatory cytokines (Saemann et al., 2000). Butyrate reduces the expression of 16 genes that codify cytokine signaling pathways, particularly that of interferon-gamma (IFNg). The anti-inflammatory properties of butyrate are owed to its interference in cytokine signaling pathways. It is worth mentioning that SCFAs and, specifically, butyrate, bear the contrary effect on transformed (cancerous) cells in culture, inducing, rather than suppressing, apoptosis. The ability of butyrate to induce apoptosis in cancerous cells may contribute to the anti-cancer properties of fiber in the diet.

Whatever the case may be, it should be emphasized that a positive anti-inflammatory effect that butyrate bears on specific points in the digestive tract does not imply a beneficial effect for the relief of GERD symptoms because, contrary to colon enterocytes, it has not been described that SCFAs and, specifically, butyrate, are used as energy substrates for cell strains of the upper digestive tract. Logically, the changes in and conformation of the digestive system clearly differentiates between those parts of the system exposed to the continuous presence of SCFAs in significant and constant concentrations and those parts that are not. Bacterial colonization of the digestive system is almost exclusively located in the cecum and the colon, and these are the only locations in which SCFAs are produced, through anaerobic fermentation. Colonocytes are therefore adapted to the medium and they are in constant contact with the SCFAs produced through fermentation. It is therefore logical that colonocytes have developed mechanisms that allow them to positively use SCFAs and, specifically, butyrate, within their biochemistry: energy, trophic, anti-inflammatory and anti-tumor substrate. Nevertheless, epithelial cells of the upper digestive system and, specifically, the esophagus and stomach, are not significantly exposed to SCFAs due to the nature of the medium: no substantial microbial colonization exists; neither does any fermentation process occur that naturally produces substantial quantities of SCFAs.

Obviously it can therefore not be inferred that properties of the SCFA described for colonocytes are directly applicable to any other cell line.

### Medium Chain Fatty Acids (MCFA): Caproic acid C6; Caprylic acid C8 and Capric acid C10

Medium chain fatty acids (MCFA) and, specifically, caproic acid C6, caprylic acid C8 and capric acid C10, are described as molecules with anti-microbial and anti-yeast net effects. Specifically, caprylic acid is used in the treatment of certain bacterial infections in dairy ruminants¹⁷. As a result of its relatively short carbon chain, it has no difficulty in penetrating the lipid bilayer of cell membranes of certain bacteria that possess lipid membranes, such as *Staphylococcus aureus* and various species of *Streptococcus¹⁸.*

Caprylic acid is generally used as anti-microbial, disinfectant and generally fungicide¹⁹ in all kinds of industries (Antimicrobials Division - www.regulations.gov - Docket Number; EPA-HQ-OPP-2008-0477 Caprylic (Octanoic) Acid).

Medium chain fatty acids (MCFA) are often used in patients suffering from Crohn's Disease (CD) or from symptoms of colitis. Sufficient studies have still not been done on their anti-inflammatory action on the intestine. A published work reports that MCFA and, specifically, caprylic acid, inhibit IL-8 secretion, at gene transcription level, in Caco-2 cells precultured with MCT for 24 hrs.²⁰ IL-8 is an α-chemokine with some potent chemo-attractive neutrophil properties and activating properties (Oppenheim et al., 1991; Baggiolini et al., 1989) and it is one of the most important chemokines in the irritable colon pathogenesis. Ulcerous colitis (UC) and Crohn's disease (CD) patients show high levels of it in intestinal mucosa; it is a good marker for assessing the effectiveness of the therapies (Mazzucchelli et al., 1994).

Other important uses of MCFAs are as surfactant and foaming agents. Unlike esters from long chain fatty acids, MCFA esters, particularly those of C6, C8 and C10, from monopropylene glycol, ethylene glycol, glycerol, etc., are water miscible, and they create stable micelles, thus producing foams with increased volume and persistence. Propylene glycol diester from caprylic and capric acids, marketed under the MIGLYOL 840 trademark, is specifically used for that purpose. The moderate use of MCFA derivatives, especially in the form of esters, provides the product of the invention the capacity to generate a certain amount of foam that may help protect the esophagus from coming into contact with the acid reflux.

### Garlic (allium sativum)

For thousands of years garlic has been used for its medicinal properties. Since the XIX century, much research has led to the establishment of an extensive series of properties including antibacterial, antiviral, antifungal, antiprotozoal, anti-inflammatory, etc., as well as beneficial effects on the immune and on the cardiovascular systems.

Garlic (*allium sativum*), like onions, shallots and leeks, among others, belongs to the alliaceae family. They all contain organosulfur products (OSC). Garlic in particular contains allicin, an organosulfur compound that is produced when garlic is broken or crushed, through the action of the allinase enzyme on the alliin. Allicin is a potent phytocide, with marked antibiotic and antifungal properties. The release of allicin produces other sulfur derivatives, such as ajoene, allyl sulfides, diallyl sulfides, allyl methyl disulfide, allyl methyl trisulfide, s-allyl cysteine and diallyl trisulfide. Research carried out in the XX and the XXI centuries suggests that powdered garlic can have anti-inflammatory, antimicrobial, antithrombotic, antitumor, hypolipidemic and hypoglycemic effects.

For the invention, the following are the most relevant of all the effects of the organosulfur compounds of garlic:

### 1.- Anti-inflammatory Effect:

The study of the anti-inflammatory activity²¹ of several species, measuring the inhibitor effect of the production of nitric oxide by macrophages activated through lipopolysaccharides, indicated that garlic was by far the species with the greatest anti-inflammatory power, followed by ginger, shallots, leeks and red peppers.

Specifically, its anti-inflammatory activity is mainly owed to the allicin. It is known that epithelial cells play an important role in the inflammatory processes of the intestine. A study showed that allicin pronouncedly inhibits, depending on the dosage, the spontaneous or TNF-α induced secretion of various cytokines (IL-1b, IL-8, IP-10 and MIG) from varying epithelial cell lines, suppressing the expression of interleukin 8 (IL-8) and interleukin 1b (IL-1b) mRNA. These observations proved that allicin exercises an immunomodulator inhibiting effect on epithelial cells, and could therefore be capable of attenuating intestinal inflammation²².

In animal models²³, low doses of garlic oil could suppress iNOS (inducible nitric oxide synthase) activity, ulceration and apoptosis of the intestinal mucosa in rats injected with endo-toxic agents. At high doses, garlic oil showed toxic effect, which is why it is deduced that garlic is beneficial in moderate doses, but can be toxic in high or very high doses.

Recent studies²⁴ verify the effect of non-steroidal anti-inflammatory agents, such as ibuprofen, in inhibiting the migration of leukocytes through the monolayers of endothelial cells. Neutrophils play an important role during an inflammation process. They migrate rapidly from the vascular space up to the tissues to destroy the invading microorganisms. Garlic extract seems to bear the same effect as that of ibuprofen, slowing down the migration of neutrophils through endothelial cell layers.

Whatever the case may be, again, and as has already been mentioned in the case of butyric acid, the fact that garlic has a positive anti-inflammatory effect on certain points of the digestive tract does not imply a beneficial effect for relieving GERD symptoms because, paradoxically, the medical community has identified garlic as a risk factor for detonating episodes of GERD (Robert D. Mootz, Howard T. Vernon "Best Practices in Clinical Chiropractic", page 44, 1999) since it reduces LES pressure and favors reflux (John C. Babka and Donald O. Castell, "On the genesis of heartburn", Am. J Dig Dis. 1973; 18:391). A recent work published in 2006 in Clinical Reviews, Richard H.Davis Jr, PA-C, Mary Knudtson, DNSc, NP, and Eugene A.Oliveri, DO, MSc "Treatment Options for the Patient With Frequent Heartburn", 2006, pages 4 and 7, continues to identify garlic and onions as causing heartburn.

### 2. Antiseptic, antibiotic²⁵, antimicotic and vermifuge activity

Many studies have confirmed the phytocidal properties²⁶ of garlic. Among all, the latest studies carried out on the effect it bears on *Helicobacter pylori²⁷* should be highlighted. *Helicobacter pylori (H. pylori)* is a bacteria involved in the etiology of stomach ulcers and of stomach cancers.

Alliaceae, and in particular, garlic, show antibiotic properties against gram-positive and gram-negative bacteria. Published studies have concluded as follows: 1) that garlic juice is effective against a large amount of intestinal bacterial pathogens responsible for diarrhea in humans and animals; 2) that garlic is effective even against certain bacterial strains resistant to antibiotics; 3) that the combination of garlic with antibiotics yields mixtures of partial or total synergy; 4) that it has repeatedly been observed that there is an absence of bacterial resistance to garlic and of development of bacterial resistance to garlic; 5) and that garlic seems to prevent the production of bacterial toxins²⁸.

### 3.- Antitumor activity

Although the most recent studies seem to indicate that the effectiveness of garlic OSCs is merely preventive but does not cure the various tumors, said preventive effect is, however, very potent. According to the compiling work of the important book, *Modern Nutrition in Health and Disease²⁹* OSCs employ two convergent means to inhibit the appearance of tumors:

### a) Induction of cytochrome P450 enzymes

Cytochrome P450 contains a group of microsomal enzymes found in most of the tissues but which are predominant in the liver.

Their function in cells is to assist in rapid detoxification and excretion of metabolic waste that could affect the cell differentiation process. Garlic OSCs help in and boost this detoxifying action of P450 enzymes.

On the other hand, P450 synergizes with the Glutathione S-transferase (GST) enzyme. When this is activated by garlic OSCs, the GST helps its substrate (glutathione) to conjugate with the toxic species produced by the enzymatic activity of P450. Thus, these metabolites are less harmful to the cell and are excreted much quicker.

### b) Glutathione S-transferase (GST) enzyme activation

GST activity was measured after the application of chemical carcinogenic agents to healthy tissues. Upon the application of garlic OSCs (diallyl disulfide and triallyl disulfide) GST activity increases remarkably and the incidence of tumors diminishes.
Glutathione (GST substrate) exercises its tumor inhibition action by conjugating with carcinogenic compounds and deactivating their capacity to bind to the cell DNA and prevent the commencement of cell damage.

In models with mice transplanted with various tumors, it has been proven that garlic activity is significant orally, but not intraperitoneally.

Epidemiological studies and experimental data confirm the effect of alliaceae in the prevention of intestinal cancer³⁰. Inducible cyclooxygenase (COX-2) isoforms are linked to the pathogenesis of a number of inflammatory diseases and to cancer processes and, specifically, to gastric tumors. Ajoene, an organosulfur component of garlic, proved it was able of inhibiting COX-2 by applying a mechanism very similar to other compounds such as indomethacin (non-steroidal anti-inflammatory drug)³¹.

### Curcuma

Curcuma is the common name for *Curcuma domestica Loiror Curcuma longa L*. The rhizomes of the plant are used as spice and for the preparation of extracts. Curcuma contains a substance referred to as curcumin, which possesses antioxidant and anti-inflammatory effects. Its anticancer effects have also been proven.

The effects of powdered curcuma are beneficial to the stomach. It increases secretion of mucin in rabbits and can therefore act as gastroprotector against irritant substances. There is, however, some controversy on the antiulcer activity of curcumin: both antiulcerative³² and ulcerative effects³³ of curcumin have been described, and today there is a research vacuum on the issue.

Curcumin has been proven to protect the stomach against the ulcerogenic effects of phenylbutazone (Bute) and 5-hydroxytryptamine (5-HT) in Guinea pigs. Its use did not, however, prevent ulcers caused by histamines in the stomach³⁴. As a matter of fact, at dosages of 50 mg/kg and 100 mg/kg, it caused ulcers in rats³⁵. Although the mechanism has not been completely elucidated, an increase in acid or pepsin secretion and a reduction in the gastric mucin layer seem to be causes of the gastric ulcers that appear³⁶. If curcuma and/or curcumin seem to increase mucin secretion in the stomach, the causes of gastric ulcer may be avoided with the prophylactic administration of this substance. Recent studies indicate that curcumin may block gastric ulcer caused by indomethacin, alcohol or stress.³⁷

### Examples

### Example of preparation of a preferred composition of the invention:

One 500 mg net weight capsule contains the following composition:
- Buffered butyric acid: 450 mg. Out of this 450 mg, 380 are of dry matter, and out of those 380 mg, 50 to 58% are sodium butyrate, and the rest, up to 100%, are various phosphates and carbonate salts in varying proportions.
- Dehydrated, powdered garlic: 50 mg, with an average water content of between 10 and 25%.
- Magnesium stearate: 0.1 to 0.5 mg.

The components are mixed in a Lödige-type high-speed paddle mixer for at least three minutes at a speed of 1500 rpm. The mixture is made based on the proportions indicated in the formula.

As an option, and in order to reduce the particle size to less than 1 mm, choppers may be set to between 1500 and 3000 rpm during the last minute of the mixing. The mixture is then emptied from the mixer on to a <1 mm sieve to be sifted until the product is left with a particle size distribution of 40% or less per a 200 micron sifter, and 100% percent passage through a 1 mm sifter. The sifted product is mixed with the amount of encapsulation coadjuvants selected, preferably in not more than 0.5% by weight of the final mixture. This is subsequently followed by the automatic encapsulation, into vegetable capsules, and their bottling and packing process.

### Dosage

The effective dosage, both for males as well as for females, is one 500 mg capsule in the morning and another at either lunch or dinner, that is, a total of 2 (two) capsules daily. However, there are volunteers who only require one capsule per day, while others double the dosage when they engage in excesses that are often the triggering factors of episodes of heartburn, like in the case of abundant meals and/or excess alcohol intakes. Dosage units should be preferably administered before meals, specifically at least 30 to 90 minutes before lunch, and preferably 30 to 60 minutes before lunch.

### Product Stability

The compositions of the invention continue to have equal effectiveness over time. The oldest samples that have been kept as reference samples for over two (2) years have been administered in blind trials to various subjects without there being significant differences as regards their effectiveness and action. Analytical study of the old samples shows the concentration of butyric acid to be of the same significance as that of the original. Nevertheless, there is evidence of a change in allicin concentration towards other more stable derivative products (s-allylcisteine and s-allylmercaptocisteine). These data concur with the data from other researchers on what is referred to as "aged garlic extract" or "AGE"³⁸.

### Examples of studies using volunteers

The invention has been tested on close to 120 volunteers who were undergoing chronic treatments with PPIs, H2 antagonists or antacids and up to the present, the results have been mostly positive. Most of the volunteers were able to replace their medication based on PPIs, H2 antagonists and antacids. Many of them noticed an increase in their quality of life and they reported that the new product (in the proposed formulations) allowed them to once again ingest moderate amounts of alcohol, spicy foods and other foodstuffs that, under normal conditions, would have caused symptoms of stomach malaise and pain. A high percentage of volunteers also reported improvements in intestinal comfort, especially in bowel movement, something that was not occurring while on other medications. Many subjects reported that the use of the product helped them achieve better digestion of their meals, allowing them to increase quantity without suffering indigestions or heartburn.

An efficiency and replacement pilot blind study was carried out in a group of volunteers. For a period of 12 months, volunteers were chosen preferably from among those with a clinical history that included diagnosis of GERD, chronic heartburn, esophagitis or any of their symptoms outlined above. Likewise, volunteers also suffering from lower digestive system dysbiosis, like colitis, ulcerous colitis, spasmodic colitis, or Crohn's disease, were assessed. The volunteers were subdivided based on the medications they used for their symptoms: none, antacids, H2 antagonists and PPIs. The study was done on 50 adult individuals: 35 men and 15 women, with ages ranging between 31 and 73 years old, with an average of 52 ± 7 (SD).

The protocol consisted in replacing the medication that each was being administered with a daily dose of 1000 mg, divided into 2 capsules of 500 mg each. One capsule was administered approximately 30 minutes before breakfast and the other, as the patient preferred, 30 minutes before the next main meal: lunch or dinner. They were allowed to increase the dosage up to a maximum of double the recommended dose, depending on their life style: social events, alcohol intake and spicy food consumption. The trial lasted for at least six weeks, followed by quarterly monitoring for as long as the volunteer agreed to continue collaborating, for up to a maximum period of 24 months. The first six weeks were divided into three periods of two weeks each. In each period one of the three formulations was tested. The formulas (damp base and initial formula) tested were:
Formula I - Buffered Na butyrate, 90%, dehydrated, powdered garlic, 10%
Formula II - Buffered Na butyrate, 60%. Mixture of butyric, capric and caprylic acid glycerin esters, 30% (butyric acid glycerol triester, 85%, capric and caprylic acid glycerol esters, 15%), dehydrated, powdered garlic, 5%, curcuma extract, 5%
Formula III - Mixture of butyric, capric and caprylic acid glycerin esters, 95% (butyric acid glycerol triester, 85%, capric and caprylic acid glycerol esters, 15%), curcuma extract, 5%

Of the three formulations proposed, the study always started with Formula I. Subsequently, regardless of the result obtained, two weeks later Formula I was always replaced with Formula II. Finally, two weeks later Formula II was replaced with Formula III. Out of the whole number of subjects studied only 2 willingly stopped taking the treatment at the end of the first six weeks. Based on the results, follow-up was continued with the Formula deemed most effective for each individual. Success was defined in the trials if the volunteer reported that he/she did not suffer any symptoms (mainly dyspepsia) during the treatment, and that his/her quality of life was the same or better than what it had been before the trial. Table 1 shows the success rate in the total mitigation of the symptoms associated with GERD:

**Table 1: % success in the relief of GERD-associated symptoms, without supplementary medication.**

| Condition | Number of Males/Females | Groups | | |
|---|---|---|---|---|
| | | Formula I | Formula II | Formula III |
| Heartburn | 7/3 | 100%/67% | 85%/66% | 57%/33% |
| Diagnosed GERD | 5/2 | 80%/100% | 60%/50% | 40%/50% |
| GERD + Colitis | 15/6 | 87%/83% | 66%/66% | 53%/33% |
| GERD + Crohn | 2/1 | 50%/100% | 50%/0% | 50%/0% |
| GERD + dysbiosis | 5/4 | 100%/100% | 60%/50% | 40%/25% |

The following cases may be mentioned as examples:

### Example 1

63 year old male patient, diagnosed with GERD 10 years before. Continuously treated with 20 mg of PPI daily since he was diagnosed; surgical operation on the vocal cords due to edema caused by the aspirations due to chronic stomach acidity. Treatment successfully replaced with the recommended (Formula I) dosage of the invention at the end of the six weeks. As prevention, his dose was doubled before social events with risk factors but he did not suffer any episode of heartburn.

### Example 2

55 year old male patient. Continuously treated with 75 mg of H2 antagonists daily for the previous 15 years; the blocker was replaced with two capsules of the product of the invention (Formula I) to be taken daily after the first six weeks without suffering any marked degradation in life quality. As prevention, dosage is doubled on days with social events, to pave the way for the intake of strong alcohol; the results are variable. The maintenance dosage is effective, and the H2 antagonist treatment was abandoned; placed under supervision for two months.

### Example 3

36 year old male patient. Continuously treated with 20 mg of PPI daily for the previous 5 years; professional basketball player, suffers from common episodes of regurgitation during training sessions. The daily and exclusive use of a 500 mg capsule (Formula I) after the initial period, reduces the episodes of pyrosis during physical efforts. Patient also states that the bowel movement improved remarkably and became regular; stools became less watery, better formed, and changed in both color and smell. Patient continued with the treatment for 15 months without reporting any secondary effects or negative changes.

### Example 4

36 year old male patient; Continuously treated with 75 mg of PPI daily for the previous 10 years; diagnosed with GERD and spasmodic colitis; worked as sales representative, which forced him to live on a diet rich in fats, proteins, spices, alcohol, etc., but very poor in fiber, fruits and vegetables. He prefers sedentary life, he is obese and a smoker. He responds to the treatment in all variations of the formulation administered (Formula I, Formula II and Formula III). PPI treatment was discontinued. In spite of the PPI's used to treat GERD symptoms, he still suffered intestinal spasms throughout the three hours following each meal. The spasms are repeated, with short and watery bowel movements. The subject responded very positively to the product of the invention, reporting great reduction in the episodes of colitis. The previous treatment was replaced with that of the invention (Formula I + Formula III) without patient reporting any secondary effects. Patient was supervised for 12 months.

### Example 5

40 year old female patient undergoing heartburn treatment with antacids and foaming agents. She was diagnosed with nervous colitis when she was 30. The daily use of the invention (Formula III) regularized her bowel movement and reduced the episodes of colitis. The heartburn episodes were mitigated to the point that the patient classified them as "very infrequent occasional events, with long periods without recurrence".

### Example 6

72 year old male patient, the first of the subjects to report the original effect of the product (Formula I). Former employee of a multinational pharmaceutical company, with knowledge and experience in detecting the original effects not previously described. He has been undergoing heartburn treatment with medication for the last 25 years. Said treatment was successfully replaced with that of the product of the invention. He reported that, for the first time in 25 years, he has been able to moderately consume wine at meals without suffering any intense episodes of heartburn (dyspepsia) the next day. He definitely rejects his chronic treatment with PPIs for that of the product of the invention as detailed in Formula I. He remains on the treatment for 24 months. On the 17^{th} month, the formulation is changed to Formula III. He responds negatively, with a relapse of the episodes of dyspepsia, although partially attenuated. He requests reinstatement of the original treatment (Formula I). For 24 months the subject has been administered the originally manufactured Formula I. Tests showed that the product aged (see AGE) in the capsules, but the volunteer did not report any loss of effectiveness during the treatment. When the treatment was discontinued after 24 months, the symptoms reappeared in approximately two weeks, and Formula I treatment had to be continued.

### Continued use, toxicity, tolerance and palliative use

A group of volunteers consumed daily doses of the product of the invention (mainly Formula I and Formula III) for a period of nearly 24 months, without referring any adverse effects, intolerance or crossed effects with other medications (which have not been studied in-depth). The subjects of the study have not shown any signs of reduction in the effectiveness of the product (Formulas I, II and III), or of need to gradually increase the doses to maintain the desired effects, meaning that there is no development of tolerance. When the product of the invention was discontinued, patients' GERD symptoms slowly reappeared. The conclusion is therefore that the effectiveness of the product is palliative, not curative, since there is no evidence of reversing the underlying causes of GERD, as may be the excessive relaxation of LES.

### Dependent side effects

Among other things, acid production in the stomach has two essential functions:
1. Antiseptic barrier: to prevent the passage of external pathogens into the intestine. Gastric acid, composed mainly of hydrochloric acid (HCl), has a very low estimated pKa of -8³⁹ although it is accepted that its acidity is 0 even in diluted solutions. This acidity is lethal for most of the bacteria and viruses swallowed through the mouth with food.
2. Effective digestion of food: the stomach is one of the main points of digestion of food. Gastric juices and various enzymes mix up in the stomach. One of them is pepsin, an enzyme that degrades proteins. It hydrolyzes proteins in their simplest parts, known as peptides. Pepsin forms in the stomach, in the presence of hydrochloric acid, from pepsinogen.

Therefore, maintaining the normal and efficient production of stomach acid helps execute these very important functions. The following occur if the stomach acid is normal:
a.- The natural barrier against pathogens becomes effective.
b.- Food digestion becomes more efficient and effective.

Although no consensus exists on long-term effects of the use of PPIs or H2 antagonists in the treatment of GERD symptoms, a current of opinion suggests the hypothesis that their use may, with time, lead to digestive and/or poor absorption disorders. Recent studies do not seem to encounter a cause-effect relationship between the continuous use of PPIs and the digestive disorders of the lower tract. This fact has, however, not been altogether proven. In view of doubts, and the lack of more data, it must be stated that since the compositions of the invention do not reduce stomach acid production, they do not interfere with the natural functions of the stomach, rather they allow it to function normally and to fulfill its functions within the digestive process. Therefore, the compositions of the invention should not be clouded by the controversy that seems to provoke the chronic use of other compounds, such as PPIs.

On the other hand, although there is no confirmation of any connection, it is no secret that some GERD patients also report lower digestive tract disorders or dysbiosis. These disorders often appear in the form of alternations between episodes of repetitive and spasmodic diarrhea, and episodes of constipation. The smell of the feces is often pungent and the color is dark (most probably due to the presence of putrescine-, cadaverine, histamine and skatole-type biogenic amines, derived from abnormal putrefactions) commonly accompanied by gases. These disorders can be classified as colitis, ulcerative colitis, irritable colon syndrome, diverticulitis, Crohn's disease, etc. All of them usually bear marked characteristics of inflammation. The subjects treated with the compositions of the invention, who, in addition to GERD, also suffered episodes of intestinal dysbiosis, stated that using the product, their digestive system and, in particular, the distal or colon digestive tract, felt gradually better in general. One subject specifically claimed that his intestinal comfort got considerably better with the continuous use of the product, in comparison with the use of PPIs, which, even though they relieved the symptoms of heartburn, failed to prevent the symptoms of spasmodic colitis.

### References

1 Prevalence of Gastroesophageal Reflux Disease: 3-7% of the population in the USA 1985 for "GERD and related esophageal disorders" (Digestive diseases in the United States: Epidemiology and Impact - NIH Publication No. 94-1447, NIDDK, 1994) Prevalence Rate: approx 1 in 33 or 3.00% or 8.2 million people in USA. Prevalence of Gastroesophageal Reflux Disease: 3 to 7 percent of U.S. population (1985) (Source: excerpt from Digestive Diseases Statistics: NIDDK)
2 http://jiag.org/Jiag/managementofdyspepsia.pdf
3 "Inhibition of Helicobacter pylori by garlic extract (Allium sativum)" Luigina Cellini, Emanuela Di Campli, Michele Masulli, Soraya Di Bartolomeo and Nerino Allocati. Instituto di Medicina Sperimentale, Facoltà di Medicina e Chirurgia, Università G.D'Annunzio, Via Dei Vestini 31, Chieti, Italy
4 Kim, Young-In, "Short-Chain Fatty Acids in Ulcerative Colitis", NUTRITION REVIEWS, Vol. 56, 1, pp. 17-24
5 Soergel ,K. H., "Colonic Fermentation: Metabolic and Clinical Implications", CLIN INVESTIG 72,742-48(1994)
6 Sheppach, W., et al., "Effects of Short-Chain Fatty Acids on the Inflamed Colonic Mucosa", SCAND. J. GASTROENTEROL, Suppl. 222, pp. 53-57 (1997)
7 W. Sheppach, "Short-Chain Fatty Acids Improve Epithelia in Ulcerative Colitis? Speculation or Mechanism", Falk Symp. (1994),73 (Short Chain Fatty Acids), pp. 206-213; diversion colitis, Soergel, op. cit.
8 W. Sheppach, et al. "Role of Short-Chain Fatty Acids in the Prevention of Colorectal Cancer", EURJ. CANCER, Vol. 31 A, No. 7/8, pp. 1077-80, 1995
9 W. Sheppach, "Effects of Short Chain Fatty Acids on Gut Morphology and Function", GUT 1994, Supplement 1, pp. 35-38; ulcerative colitis, Jorgersen, J. R., et al., "Influence of Faeces from Patients with Ulcerative Colitis on Butyrate Oxidation in Rat Colonocytes", DIGESTIVE DISEASES AND SCIENCES, Vol. 44,10 pp. 2099-2109 (1999), Kim, op. cit., Sheppach, Falk Symp. (1994), 73 (Short Chain Fatty Acids), op. cit. (pp. 206-213)
10 Perez, R., et al. "Selective Targeting of Kupffer Cells with Liposomal Butyrate Augments Portal Venous Transfusion-Induced Immunosuppression", TRANSPLANTATION, Vol. 65,10, pp. 1294-98,1998
   Perez, R., et al. "Sodium Butyrate Upregulates Kupffer Cell PGE2 Production and Modulates Immune Function", J. SURGICAL RES., 78 pp. 1-6 (1998)
11 Watkins, S. M., et al. "Butyric Acid and Tributyrin Induce Apoptosis in Human Hepatic Tumor Cells", J. Dairy Res., 66, pp. 559-67 (1999)
12 Rabboni, G. H., et al. "Short-Chain Fatty Acids Inhibit Fluid and Electrolyte Loss Inducted by Cholera Toxin in Proximal Colon of Rabbit In Vitro", DIGESTIVE DISEASES AND SCI., Vol. 44, 8, pp. 1547-53 (1999)
13 Acta Vet Hung. 1990;38 (1-2):3-17. "Feeding trial in pigs with a diet containing sodium n-butyrate" Gálfi P, Bokori J. Department of Physiology, University of Veterinary Sciences, Budapest, Hungary
14 "Performance, intestinal microflora, and wall morphology of weanling pigs fed sodium butyrate." Biagi G, Piva A, Moschini M, Vezzali E, Roth FX
15 W. Frankel et al., "Butyrate Increases Colonocyte Protein Synthesis in Ulcerative Colitis" Journal of Surgical Research, 57, pp. 210-214 (1994)
   A. Finnie et al., "Colonic Mucin Synthesis is Increased by Sodium Butyrate", Gut, 36, pp. 93-99 (1995)
16 "Effects of intravenous injection of butyrate on the exocrine pancreatic secretion in guinea pigs" K. Katoh a. and T. Tsudaa
17 "Antibacterial Effect of Caprylic Acid and Monocaprylin on Major Bacterial Mastitis Pathogens" M.K.M. Nair1, J. Joy1, P. Vasudevan1, L. Hinckley2, T.A. Hoagland1 and K.S. Venkitanarayanan
18 Nair MK, Joy J, Vasudevan P, Hinckley L, Hoagland TA, Venkitanarayanan KS (Oct 2005). "Antibacterial effect of caprylic acid and monocaprylin on major bacterial mastitis pathogens". J Dairy Sci 88 (10):3488-95
19 "Food and Nutrients in Disease Management" Ingrid Kohlstadt pp. 166, 169
20 "Caprylic acid and medium-chain triglycerides inhibit IL-8 gene transcription in Caco-2 cells: comparison with the potent histone deacetylase inhibitor trichostatin A" Aihiro Hoshimoto, Yasuo Suzuki, Tatsuro Katsuno, Hiroshi Nakajima and Yasushi Saito
21 "Antioxidant and anti-inflammatory activities of several commonly used spices." Tsai TzungHsun, Tsai PoJung, Ho SuChen
22 "Allicin inhibits spontaneous and TNF-alpha. induced secretion of proinflammatory cytokines and chemokines from intestinal epithelial cells" Lang, Alon; Lahav, Maor; Sakhnini, Emad; Barshack, Iris; Fidder, Herma H.; Avidan, Benjamin; Bardan, Eitan; Hershkoviz, Rami; Bar-Meir, Simon; Chowers, Yehuda. Clinical Nutrition (2004), 23(5), 1199-1208 CODEN: CLNUDP; ISSN: 0261-5614
23 "Effects of garlic oil and two of its major organosulfur compounds, diallyl disulfide and diallyl trisulfide, on intestinal damage in rats injected with endotoxin"
24 Middle East J Anesthesiol. 2000 Oct;15(6):649-58. "Garlic extract (allium sativum) reduces migration of neutrophils through endothelial cell monolayers." Hobauer R, Frass M, Gmeiner B, Kaye AD, Frost EA
25 In Vitro and In Vivo Activities of Garlic against Helicobacter pylori. Roe IH, Nam SW, Myung NH, Kim JT, Shin JH
26 Rev Iberoam Micol. 2006 Jun;23(2):75-80."Ajoene the main active compound of garlic (Allium sativum): a new antifungal agent"
27 "Activities of Garlic Oil, Garlic Powder, and Their Diallyl Constituents against Helicobacter pylori" E. A. O'Gara, D. J. Hill, and D. J. Maslin
28 "Protection against Helicobacter pylori and Other Bacterial Infections by Garlic" Gowsala P. Sivam
29 "Modern Nutrition in Health and Disease" Maurice E. Shils (Editor), James A. Olson (Editor), Moshe Shike (Editor), A. Catherine Ross (Editor). Lippincott Williams & Wilkins; 9th edition
30 "Garlic: A Review of Its Medicinal Effects and Indicated Active Compounds" Phytomedicines of Europe Chapter 14, pp 176-209
31 "Ajoene, a natural product with non-steroidal anti-inflammatory drug (NSAID)-like properties?" Verena M. Dirsch and Angelika M. Vollmara. Institute of Pharmacy, Center of Drug Research
32 "Study of the mechanism of action of curcumin; an antiulcer agent." Sinha, M., Mukherjee, B. P., Mukherjee, B., Sikdar, S. and Dasgupta,S. R. Indian J. Pharmacol., 1975, 7, 98
33 "Studies on ulcerogenic activity of curcumin." Prasad, D. N., Gupta B., Srivastava, R. K. and Satyavati, G. V., Indian J. Physiol. Pharmacol., 1976, 20, 92
34 "Effect of curcumin, its alkali salts and Curcuma longa oil in histamine induced gastric ulceration." Bhatia, A., Singh, G. B. and Khanna, N. M., Indian J. Exp. Biol., 1964, 2, 158-160
35 "Mechanisms of curcumin induced gastric ulcer in rats." Gupta, B., Kulshrestha, V. K., Srivastava, R. K. and Prasad, D. N., Indian J. Med.Res., 1980, 71, 806-814
36 "Diseases of the digestive system." Cream, G. P., Shearman, D. J. C. and Small, W. P. In Davidsons Principles and Practice of Medicine (ed. Macleod, J.) The English Language Book Society and ChurchiIILivingstone, Edinburgh, 1974, 11th ed., p. 456
37 "Curcuma and curcumin: Biological actions and medicinal applications" lshita Chattopadhyay, Kaushik Biswas, Uday Bandyopadhyay and Ranajit K. Banerjee
38 " Antioxidant Health Effects of Aged Garlic Extract" Carmia Borek, Department of Community Health and Family Medicine, Nutrition and Infectious Diseases Unit, Tufts University School of Medicine, Boston
39 Data of the Japanese Chemistry Society (1984)

## Claims

1. A composition comprising:
- at least one C1-C10 fatty acid from the group that consists of butyric acid or isobutyric acid, alone or combined, or their alkali or alkaline-earth metal salts, or a glycerol ester thereof, and
- at least one plant extract from the alliaceae family.

2. The composition of Claim 1, wherein the plant extract from the alliaceae family is a garlic extract.

3. The composition of Claim 2, wherein the garlic extract is powdered, non-deodorized, dry garlic extract.

4. The composition of any of Claims 1 to 3, wherein the alkali or alkaline-earth metal salt of at the least one C1-C10 fatty acid is a sodium, calcium or potassium salt thereof.

5. The composition of Claim 4, wherein the sodium, calcium or potassium salt of the C1-C10 fatty acid is sodium butyrate.

6. The composition of any of Claims 4 or 5, wherein the sodium, calcium or potassium salt of the C1-C10 fatty acid is buffered with a buffer selected from phosphate salts and carbonate salts.

7. The composition of any of Claims 1 to 3, wherein the glycerol ester of at least one C1-C10 fatty acid is a triglyceride thereof.

8. The composition of any one of the aforementioned Claims, further comprising a curcuma plant extract.

9. The composition of any one of the aforementioned Claims, further comprising at least one excipient selected from the group consisting of vehiculizing agents, thinners, disintegrants, lubricants, binders, gellants, flavors, sweeteners, coloring agents, preservatives, fillers, antioxidants, encapsulating coadjuvants and coating agents.

10. The composition of any one of the aforementioned Claims, which is presented in the form of capsule, pill, sachet, tablet or coated tablet.

11. The composition of any one of the aforementioned Claims for the re-establishment of digestive homeostasis affected by peptic-acid disorders and/or states of disorder caused by damage to the gastric mucosa or digestive epithelium.

12. The composition of any one of Claims 1-10 for the relief of gastroesophageal reflux or GERD Symptoms.

13. A use of a composition according to any one of the aforementioned Claims 1-10 in the manufacture of a dietary supplement.

14. A use of a composition according to any one of the aforementioned Claims 1-10 in the manufacture of a medication.

15. The use according to Claim 14, wherein the medication is for the re-establishment of digestive homeostasis affected by peptic acid disorders and/or states of disorder caused by damages to the gastric mucosa or digestive epithelium such as gastroesophageal reflux or GERD.

## Patentansprüche

1. Zusammensetzung, die
- mindestens eine C1-C10-Fettsäure aus der Gruppe, die aus Buttersäure oder Isobuttersäure, allein oder in Kombination, oder ihren Alkalimetall- oder Erdalkalimetallsalzen oder einem Glycerinester davon besteht, und
- mindestens einen Pflanzenextrakt aus der Familie der Zwiebelgewächse umfasst.

2. Zusammensetzung nach Anspruch 1, worin der Pflanzenextrakt aus der Familie der Zwiebelgewächse ein Knoblauchextrakt ist.

3. Zusammensetzung nach Anspruch 2, worin der Knoblauchextrakt ein pulverförmiger, nicht desodorierter, trockener Knoblauchextrakt ist.

4. Zusammensetzung nach einem der Ansprüche 1 bis 3, worin das Alkalimetall- oder Erdalkalimetallsalz der mindestens einen C1-C10-Fettsäure ein Natrium-, Calcium- oder Kaliumsalz davon ist.

5. Zusammensetzung nach Anspruch 4, worin das Natrium-, Calcium- oder Kaliumsalz der C1-C10-Fettsäure Natriumbutyrat ist.

6. Zusammensetzung nach einem der Ansprüche 4 oder 5, worin das Natrium-, Calcium- oder Kaliumsalz der C1-C10-Fettsäure mit einem Puffer, der ausgewählt ist aus Phosphatsalzen und Carbonatsalzen, gepuffert ist.

7. Zusammensetzung nach einem der Ansprüche 1 bis 3, worin der Glycerinester der mindestens einen C1-C10-Fettsäure ein Triglycerid davon ist.

8. Zusammensetzung nach irgendeinem der vorangehenden Ansprüche, die weiterhin einen Kurkuma-Pflanzenextrakt umfasst.

9. Zusammensetzung nach irgendeinem der vorangehenden Ansprüche, die weiterhin mindestens einen Hilfsstoff, der ausgewählt ist aus der Gruppe bestehend aus Trägerstoffen, Verdünnungsmitteln, Sprengmitteln, Gleitmitteln, Bindemitteln, Gelbildnem, Aromen, Süßstoffen, Farbstoffen, Konservierungsmitteln, Füllstoffen, Antioxidantien, Verkapselungshilfsstoffen und Überzugsmitteln, umfasst.

10. Zusammensetzung nach irgendeinem der vorangehenden Ansprüche, die in Form von Kapsel, Pille, Beutel, Tablette oder überzogene Tablette vorliegt.

11. Zusammensetzung nach irgendeinem der vorangehenden Ansprüche zur Wiederherstellung des Verdauungsgleichgewichts, das durch säurebedingte Erkrankungen des Magens und/oder durch Krankheitszustände, die durch Schädigung der Magenschleimhaut oder des Verdauungsepithels verursacht werden, gestört ist.

12. Zusammensetzung nach irgendeinem der Ansprüche 1 bis 10 zur Linderung der Symptome des gastroösophagealen Reflux oder GERD.

13. Verwendung einer Zusammensetzung nach irgendeinem der vorangehenden Ansprüche 1 bis 10 bei der Herstellung eines Nahrungsergänzungsmittels.

14. Verwendung einer Zusammensetzung nach irgendeinem der vorangehenden Ansprüche 1 bis 10 bei der Herstellung eines Medikaments.

15. Verwendung nach Anspruch 14, wobei das Medikament zur Wiederherstellung des Verdauungsgleichgewichts eingesetzt wird, das durch säurebedingte Erkrankungen des Magens und/oder durch Krankheitszustände, die durch Schädigung der Magenschleimhaut oder des Verdauungsepithels verursacht werden, wie dem gastroösophagealen Reflux oder GERD, gestört ist.

## Revendications

1. Une composition comprenant :
- au moins un acide gras C1-C10 du groupe comportant acide butyrique ou acide isobutyrique, seul ou combiné, ou leurs sels métalliques alcalins ou alcalino-terreux, ou un ester glycérique du même acide gras C1-C10,
et
- au moins un extrait de plante de la famille des alliacées.

2. La composition de la revendication 1, dans laquelle l'extrait de plante de la famille des alliacées est un extrait d'ail.

3. La composition de la revendication 2, dans laquelle l'extrait d'ail est un extrait d'ail sec, en poudre, non désodorisé.

4. La composition de n'importe laquelle des revendications 1 à 3, dans laquelle le sel métallique alcalin ou alcalino-terreux d'au moins un acide gras C1-C10 est un sel de sodium, de calcium ou de potassium de ce dernier.

5. La composition de la revendication 4, dans laquelle le sel de sodium, de calcium ou de potassium de l'acide gras C1-C10 est du butyrate de sodium.

6. La composition de n'importe laquelle des revendications 4 ou 5, dans laquelle le sel de sodium, de calcium ou de potassium de l'acide gras C1-C10 est tamponné avec un tampon choisi parmi des sels de phosphate et des sels de carbonate.

7. La composition de n'importe laquelle des revendications 1 à 3, dans laquelle l'ester glycérique d'au moins un acide gras C1-C10 est un triglycéride de ce dernier.

8. La composition de n'importe laquelle des revendications susmentionnées, comprenant en outre un extrait de plante de curcuma.

9. La composition de n'importe laquelle des revendications susmentionnées, comprenant en outre au moins un excipient sélectionné dans le groupe composé d'agents véhicules, diluants, délitants, lubrifiants, liants, gélifiants, arômes, édulcorants, colorants, conservateurs, agents de remplissage, antioxydants, adjuvants d'encapsulation et agents d'enrobage.

10. La composition de n'importe laquelle des revendications susmentionnées, qui est présentée sous forme de gélule, pilule, sachet, comprimé ou comprimé enrobé.

11. La composition de n'importe laquelle des revendications susmentionnées destinée à rétablir l'homéostasie digestive affectée par des troubles d'acidité peptique et/ou des états pathologiques dus à la lésion de la muqueuse gastrique ou de l'épithélium digestif.

12. La composition de n'importe laquelle des revendications 1-10 pour la suppression des reflux gastro-oesophagiens ou des symptômes de GERD (reflux gastro-oesophagiens pathologiques).

13. Une utilisation d'une composition selon n'importe laquelle des revendications 1-10 susmentionnées dans la fabrication d'un complément alimentaire.

14. Une utilisation d'une composition selon n'importe laquelle des revendications 1-10 susmentionnées dans la fabrication d'un médicament.

15. L'utilisation selon la revendication 14, dans laquelle le médicament est destiné à rétablir l'homéostasie digestive affectée par des troubles d'acidité peptique et/ou des états pathologiques dus à des lésions de la muqueuse gastrique ou de l'épithélium digestif comme les reflux gastro-oesophagiens ou GERD.
